# DEMANDE DE BREVET EUROPEEN

(11) **EP 0 536 458 A1**
(43) Date de publication de la demande: **14.04.1993**
(21) Numéro de dépôt: 91440112.0
(22) Date de dépôt: 27.12.1991
(51) Int. Cl.: A61F 13/58

(54) **Couche-culotte**

(30) Priorité: 12.09.1991 FR 9111525
(71) Demandeur: PROTECO Sarl, F-59178 Hasnon (Nord) (FR)
(72) Inventeur: Brutin, Jean-Marc, F-59178 Hasnon, (Nord) (FR)
(74) Mandataire: Duthoit, Michel Georges André

(57) **Abrégé**

Couche-culotte destinée notamment pour enfants en bas âges ou pour personnes incontinentes comprenant au moins une feuille imperméable (2) externe et au moins deux éléments adhésifs (7) de fixation prévus au niveau des angles de la couche pour permettre le maintien de la dite couche (1) en position d'utilisation.

Selon l'invention, la couche-culotte présente une bande (12), apte à recevoir les éléments adhésifs (7), et partiellement assujettie à la face externe de la feuille imperméable (21), en ce que la partie médiane (16) de la dite bande frontale (12) est flottante afin d'autoriser une compensation de pression au niveau des éléments adhésifs lors de la fermeture de la couche-culotte.

## Description

La présente invention a pour objet une couche-culotte notamment destinée pour enfants en bas âges ou pour adultes incontinents.

Actuellement, il est connu d'utiliser des couches-culottes qui sont généralement constituées par :
- une feuille externe généralement en matière de synthèse par exemple en polyéthylène,
- une feuille interne en matériau imperméable destinée à être en contact avec la peau de la personne qui porte la couche,
- un matelas en matériau absorbant par exemple en fibres de cellulose insérées entre la feuille externe et la feuille interne.

Pour permettre le maintien en place de la couche-culotte en position adaptée, il est connu d'avoir recours à des dispositifs d'attache par adhésifs qui sont généralement fixés par collage sur au moins une partie de la face externe de la feuille imperméable et notamment sur la partie postérieure arrière.

Ces différents dispositifs d'attache sont constitués généralement par une languette adhésive fixée sur la partie arrière de la feuille externe de la couche-culotte.

Cette languette vient se fixer sur la partie avant de la feuille externe lors de la mise en place de la couche-culotte.

De tels dispositifs présentent certains inconvénients et notamment celui d'entraîner soit la déchirure de la languette, soit l'arrachement de la zone à laquelle la languette adhère lorsque l'on désire fermer plusieurs fois la couche-culotte.

C'est notamment le cas lorsque l'on désire vérifier si le matelas absorbant est mouillé ou si l'on veut remettre en position correcte la couche-culotte.

Cet arrachement de la languette ou ce déchirement de la feuille externe imperméable à laquelle la languette adhère rend alors la couche inutilisable et l'on doit alors la jeter.

Pour remédier à ces inconvénients, on connaît différentes techniques qui prévoient un renforcement de la feuille imperméable.

On connaît par exemple des couches-culottes qui comportent un dépôt de renforts dans la zone de bandes larges à l'intérieur de la feuille imperméable, ou bien des couches-culottes qui présentent des bandes de renfort à l'intérieur de la feuille imperméable notamment dans la zone de mise en place des dispositifs adhésifs.

On connaît également des couches-culottes qui présentent une zone renforcée sur la face externe de la feuille imperméable. La feuille de la dite zone renforcée est de préférence totalement lisse par opposition à la surface mâte de la feuille imperméable. Dans cette technique, la surface externe imperméable mince est traitée de façon à présenter un aspect mât qui lui donne une apparence de matériau textile en supprimant, en outre, tout effet de réflexion et de cisaillement.

L'état de surface ainsi obtenu pour la zone renforcée permet d'améliorer la résistance à la traction dans son plan de la languette collée sur la zone renforcée tout en favorisant le décollage par traction selon une direction faisant un angle avec le plan de la languette collée.

Un inconvénient majeur de ces couches-culottes réside dans leur mise en place. En effet, afin de permettre une mise en place correcte de l'adhésif, il est nécessaire d'effectuer une pression importante sur le ventre de la personne qui porte la couche, ce qui lui procure un effet désagréable, notamment pour des enfants en bas âge.

Un autre inconvénient fréquemment rencontré en pratique par ces couches-culottes réside dans le fait qu'elles ne sont pas prévues pour permettre un ajustement correct des adhésifs latéraux de fermeture de la ceinture frontale.

Par ailleurs, sans précaution d'usage, il se produit un phénomène de bâillement de la couche au niveau de la ceinture lors de la mise en place des adhésifs.

Le but de la présente invention est de proposer une couche-culotte qui permette de pallier aux inconvénients des techniques existantes.

En outre, la mise en place de cette couche-culotte est aisée, rapide et simple et elle permet d'éviter d'exercer une pression trop importante sur le ventre de l'enfant au moment de la fixation de l'adhésif.

Un des buts de l'invention est de proposer une couche-culotte dont la bande frontale à partie médiane flottante est réalisée à partir de matériaux qui présentent des propriétés de résistance, de confort et d'esthétique appréciables sans entraîner un surcoût de fabrication.

D'autres buts et avantages de la présente invention apparaitront au cours de la description qui va suivre qui n'est donnée qu'à titre d'exemple indicatif et qui n'a pas pour but de la limiter.

La couche-culotte notamment destinée pour enfants en bas âges et pour personnes incontinentes selon l'invention comprend au moins une feuille imperméable externe et au moins deux éléments adhésifs de fixation prévus au niveau des angles de la couche pour permettre le maintien de la dite couche en position d'utilisation.

Elle est caractérisée par le fait qu'elle comporte une bande, apte à recevoir les éléments adhésifs, et partiellement assujettie à la face externe de la feuille imperméable, et en ce que la partie médiane de la dite bande frontale est flottante afin d'autoriser une compensation de pression au niveau des éléments adhésifs lors de la fermeture de la couche-culotte.

L'invention sera bien comprise si l'on se réfère à la description qui suit ainsi qu'aux dessins en annexes qui en font partie intégrante.

La figure 1 est une vue schématique qui illustre une couche-culotte qui présente une bande frontale conforme à l'invention dans une position basse.

La figure 2 est une vue similaire à la figure 1 dans laquelle la bande frontale est en position haute afin de permettre le passage d'une main.

La figure 3 est une coupe selon la ligne 2.2 de la figure 2.

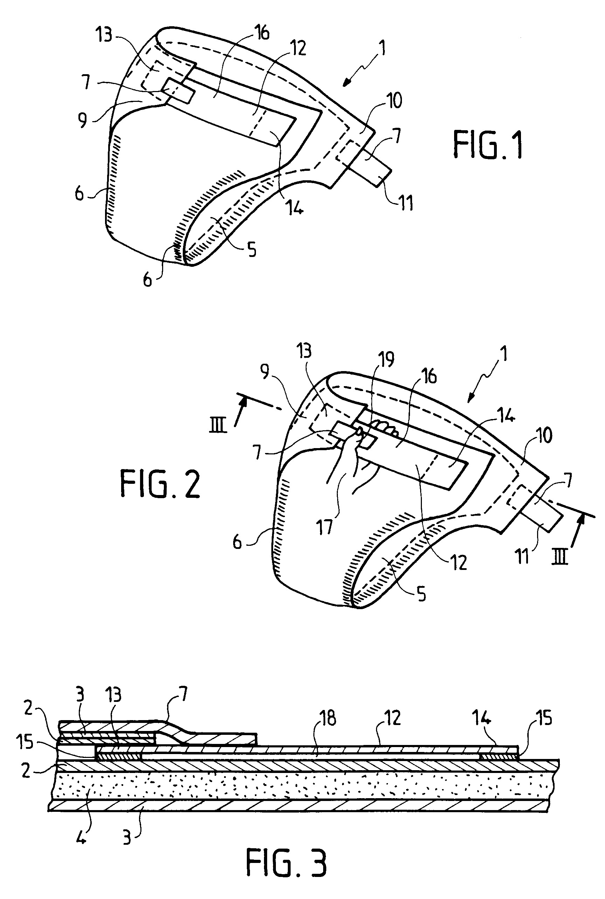

Les figures 1 et 2 représentent une couche-culotte 1 à jeter destinée notamment pour des bébés ou pour des personnes incontinentes. Cette couche-culotte 1 comprend :
- une feuille externe 2 imperméable aux liquides,
- une feuille interne 3 perméable qui vient en contact de la peau de la personne qui porte la couche,
- un tampon absorbant 4 fixé entre la feuille externe 2 et la feuille interne 3.

La feuille externe 2 et la feuille interne 3 sont généralement de dimension supérieure à celle du tampon absorbant 4 et leurs portions latérales s'étendent au delà des bords du tampon 4.

La feuille interne 3 et la feuille externe 2 sont fixées l'une à l'autre le long de leur partie latérale.

La feuille interne 3, la feuille externe 2 et le tampon absorbant 4 sont réalisés en des matériaux couramment utilisés dans le domaine des couches-culottes. Par exemple, la feuille interne 3 peut être réalisée en toutes sortes de matières tendres, flexibles et poreuses laissant en outre passer les fluides. Elle peut comprendre un voile de fibres non tissé, un papier en soie résistant à l'humidité, et une feuille de filament non tissé, etc. Cette feuille peut subir des traitements de surface afin d'accroître ses capacités de transfert des fluides notamment par des agents tensioactifs ou par tout autre agent similaire.

La feuille externe 2 est imperméable notamment aux fluides. Elle peut comprendre un voile ou une feuille fine de film en matière plastique comme par exemple en polyéthylène, polypropylène, polychlorure de vinyle ou tout autre agent équivalent. Elle peut être transparente ou présentée un aspect de surface mât ou gaufré.

Le tampon absorbant 4 est réalisé en toute matière absorbante généralement en un matériau cellulosique ou dans le cas de tampons aéroformés en fibres de bois.

L'association de la feuille externe 2 et de la feuille interne 3 au tampon 4 est réalisée par toute manière adaptée et connue de l'Homme du Métier permettant de réaliser une couche-culotte 1 complète.

C'est ainsi que les feuilles 2 et 3 et le tampon 4 peuvent être fixés les uns aux autres au moyen de bandes, ou d'éléments de forme adaptée, d'adhésifs thermosensibles, ou sensibles à la pression, de scellage à chaud, ou par toute autre technique appropriée de bandes de rubans adhésifs, etc.

La couche-culotte 1 est de forme sensiblement en I et comprend une section centrale qui présente une forme rétrécie et des zones plus élargies au niveau de la ceinture le long de chacune de ses extrémités. Des moyens élastiques 6 sont fixés en des zones adjacentes au tampon absorbant 4 de chaque côté de celui-ci pour former des portions de jambes à fronçage élastique qui puissent se conformer aux jambes du porteur de la couche afin d'éviter tout phénomène de bâillement de la couche 1 susceptible d'entraîner des fuites de liquides.

Des éléments de fixation constitués par une languette de rubans adhésifs 7 sont assujettis par des techniques classiques de collage, soudures ou toute autre technique connue de l'Homme de l'Art à chacune des extrémités 9 et 10 de la face externe de la feuille 2. Pour éviter tout collement non désiré de ces languettes, avant leur mise en place, ces languettes adhésives 7 sont revêtues d'une bande protectrice amovible 11.

La partie supérieure de la feuille imperméable 2 présente sur sa face externe avant une bande frontale 12. Cette bande frontale 12 présente une épaisseur, une longueur et une largeur variable, adaptées en fonction de l'utilisation.

Elle est réalisée dans un matériau souple et résistant. Elle s'étend sensiblement sur toute la longueur de la face externe avant de la feuille imperméable 2. Cette bande 12 constitue une bande de renforcement destinée à recevoir les languettes adhésives 7 rabattues lorsque la couche est en position d'utilisation.

C'est la raison pour laquelle elle est constituée dans un matériau qui présente un état de surface compatible avec celui des languettes adhésives 7. Elle présente une longueur sensiblement identique à celle des languettes adhésives 7.

En outre, l'existence d'une telle zone de renforcement permet d'ouvrir et de refermer la couche-culotte 1 à plusieurs reprises sans déchirer les languettes adhésives 7 ni détériorer la feuille imperméable externe 2.

Cette bande frontale 12 selon l'invention, est assujettie à chacune de ses extrémités 13, 14 par des points de colle 15 comme plus particulièrement illustré à la figure 3. Bien entendu, dans des variantes de réalisation, il est tout à fait envisageable d'assujettir chacune des extrémités 13 et 14 de la bande frontale 12 par d'autres techniques telles que par des lignes ou des pavés de colle, des points ou des lignes de soudures, par thermoscellage, ou par toute technique appropriée connue de l'Homme du Métier.

La partie médiane 16 de la bande frontale 12 est, selon l'invention, laissée libre et flottante c'est-a-dire qu'elle n'est pas fixée. Comme plus particulièrement illustrée à la figure 2, cette partie médiane 16 flottante autorise le passage d'une main 17 dans l'espace 18 ménagé entre la face avant externe de la feuille externe 2 et la bande frontale 12.

Pour faciliter le passage de la main 17 dans l'espace 18, la bande frontale 12 est assujettie à ses extrémités sur une zone de faible largeur. Ainsi, la bande frontale 12 est maintenue flottante sensiblement sur toute sa longueur.

Grâce à ce positionnement de la main 17, comme montré à la figure 2, il est possible d'exercer, lors de la mise en place de la languette adhésive 7, Une pression importante sur celle-ci sans pour autant transmettre cette pression sur le corps de la personne qui porte la couche-culotte 1. Il en résulte que la mise en place des languettes adhésives 7 sur la bande frontale 12 s'effectue rapidement sans préjudice pour le porteur de la couche puisque la pression exercée par le pouce 19 sur la languette 7 est compensée par la face de la main 17 et que de ce fait elle n'est plus transmise à la personne.

On a donc ainsi mis en évidence l'intérêt et l'utilité de cette bande frontale 12 flottante. En outre, cette compensation de la pression permet avantageusement d'éviter un bâillement de la couche-culotte 1 au niveau de la ceinture résultant de la déformation associée à la compensation de la pression exercée sur la languette adhésive 7 lors de sa mise en place.

Pour permettre une compensation encore plus efficace de la pression exercée, la mise en place de la languette adhésive 7 s'effectue en la rabattant quasitotalement sur une zone de la bande frontale 12 qui est laissée flottante comme plus particulièrement illustré à la figure 3. En position d'utilisation, les efforts exercés par les languettes adhésives 7 s'équilibrent de sorte que la région de la bande frontale 12 résiste correctement à l'allongement et à la déchirure et donne des attaches à résistance à la traction améliorée tout en étant confortable.

Dans la pratique, la bande frontale 12 à partie médiane flottante permet une mise en place rapide, aisée de la couche-culotte 1. Par ailleurs, elle facilite l'ajustement dans une position appropriée de la languette adhésive 7. Il en résulte alors un avantage incontestable qui est celui de permettre un positionnement correct et efficace de la couche-culotte 1.

Il faut également noter que selon l'invention, il est possible de réaliser des bandes frontales 12 à partir de matériaux qui présentent un état de surface compatible avec celui des éléments adhésifs 7 et qui sont mécaniquement résistants aux efforts de traction, flexion, cisaillement, etc.

De plus, ces matériaux présentent des caractéristiques de souplesse et d'élasticité qui confèrent à la couche-culotte un plus grand confort sans pour autant occasionner un surcoût de fabrication.

Il y a lieu de noter, bien entendu que l'invention s'applique à tous les types de couches-culottes 1, notamment avec ou sans élastique à la taille et/ou à l'entrejambe, qui présentent éventuellement un matelas absorbant de toutes formes s'étendant jusqu'aux bords latéraux de la feuille externe ou se terminant à une distance de ces bords avec ou sans échancrure pour les jambes, etc.

## Revendications

1. Couche-culotte notamment destiné pour enfants en bas âges et ou pour personnes incontinentes, comprenant au moins une feuille imperméable (2) externe et au moins deux éléments adhésifs (7) de fixation prévus au niveau des angles de la couche (1) pour permettre le maintien de la dite couche en position d'utilisation caractérisée en ce qu'elle comporte une bande (12), apte à recevoir les éléments adhésifs (7), et partiellement assujettie à la face externe de la feuille imperméable (2), et en ce que la partie médiane (16) de la dite bande frontale (12) est flottante afin d'autoriser une compensation de pression au niveau des éléments adhésifs lors de la fermeture de la couche-culotte (1).

2. Couche-culotte selon la revendication 1, caractérisée en ce que la bande frontale (12) est assujettie à chacune de ses extrémités (13-14) sur la face avant de la feuille externe imperméable (2).

3. Couche-culotte selon la revendication 1 caractérisée en ce que la bande frontale (12) présente une partie médiane flottante (16) s'étendant sensiblement sur toute sa longueur.

4. Couche-culotte selon la revendication 1 caractérisée en ce que la bande frontale (12) s'étend sensiblement sur toute la largeur de la partie supérieure de la feuille externe de la feuille imperméable (2).

5. Couche-culotte selon la revendication 1, caractérisée en ce que la bande frontale (12) a une largeur sensiblement identique à celle des éléments adhésifs (7).

6. Couche-culotte selon la revendication 1, caractérisée en ce que la bande frontale (12) est fixée par des points ou lignes ou pavés de colle.

7. Couche-culotte selon la revendication 1, caractérisée en ce que la bande frontale est fixée (12) par des points ou lignes de soudure à ultra-sons.

8. Couche-culotte selon la revendication 1, caratérisée en ce que la bande frontale (12) est fixée par thermo-scellage.

9. Couche-culotte selon la revendication 1, caractérisée en ce que la bande frontale (12) est réalisée en un matériau qui présente un état de surface compatible avec celui des éléments adhésifs et qui est mécaniquement résistant aux efforts de traction et de flexion.

10. Couche-culotte selon la revendication 1, caractérisée en ce que la bande frontale (12) est réalisée en un matériau souple.
